# FASCICULE DE BREVET EUROPEEN

(11) **EP 3 093 255 B1**
(45) Date de publication et mention de la délivrance du brevet: **18.10.2017**
(21) Numéro de dépôt: 16169223.1
(22) Date de dépôt: 11.05.2016
(51) Int. Cl.: B65D 43/16, B65D 8/18, B65D 85/24, A61F 15/00

(54) **BOÎTE DE CONDITIONNEMENT ET DE DISTRIBUTION DE PRODUITS, NOTAMMENT DE PRODUITS D'HYGIÈNE**
SCHACHTEL ZUR VERPACKUNG UND VERTEILUNG VON PRODUKTEN, INSBESONDERE VON HYGIENEPRODUKTEN
BOX FOR PACKAGING AND DISPENSING PRODUCTS, PARTICULARLY SANITARY PRODUCTS

(30) Priorité: 13.05.2015 FR 1554335
(43) Date de publication de la demande: 16.11.2016
(73) Titulaire: Groupe Lemoine, 92100 Boulogne Billancourt (FR)
(72) Inventeur: LEMOINE, Philippe, 75016 PARIS (FR)
(74) Mandataire: Casalonga

(56) Documents cités:
- WO-A1-93/17920
- FR-A1- 2 221 349
- FR-A1- 2 540 078
- US-A- 4 852 792
- US-A1- 2014 263 338

## Description

La présente invention concerne une boîte de conditionnement et de distribution de produits selon l'une des revendications 1 à 6. Dans une application particulièrement intéressante, l'invention concerne une boîte pour le conditionnement et la distribution de produits d'hygiène, notamment de cotons-tiges.

Les boîtes de ce type comportent classiquement un corps et un couvercle monté sur le corps de sorte que le couvercle puisse être ouvert pour prélever un produit et reste sinon fermé.

Dans divers modes de réalisation, le corps et le couvercle peuvent être réalisés en matière plastique, en particulier par moulage d'un polymère thermoplastique, en une seule pièce de sorte que le corps et le couvercle soient reliés par une charnière moulée.

Le couvercle et le corps peuvent encore être réalisés sous la forme de deux pièces distinctes, le couvercle venant alors se fixer sur le corps. Toutefois, lorsque le corps et le couvercle sont réalisés à partir de matériaux différents, des problèmes d'assemblage fiable du couvercle sur le corps peuvent alors se poser. Or, un assemblage insuffisant du couvercle sur le corps peut apparaître préjudiciable en cas de chute d'une boîte pleine, le contenu de la boîte pouvant alors se répandre au sol.

Le problème de l'assemblage fiable du couvercle sur le corps se pose de façon accrue lorsque le corps est réalisé en carton ou, de manière générale, dans un tel matériau écologiquement acceptable.

Au vu de ce qui précède, l'invention se propose d'améliorer la fixation d'un couvercle sur un corps dont le matériau est différent de celui du corps.

L'invention a donc pour objet une boîte de conditionnement et de distribution de produits, notamment de produits d'hygiène, selon l'une des revendications 1-6, comprenant notamment un corps et un couvercle monté sur le corps.

Le couvercle comporte des moyens d'encliquetage destinés à coopérer avec des moyens d'encliquetage correspondants prévus sur le corps.

On peut alors réaliser un assemblage fiable du couvercle sur le corps, indépendamment des matériaux entrant dans leur constitution, et ce, à faible coût.

Dans un mode de réalisation, le corps et le couvercle sont réalisés à partir de matériaux différents

Selon une autre caractéristique de la boîte selon l'invention, le corps comporte un fond, une paroi périphérique comprenant un ensemble de côtés latéraux dressés à partir du fond et délimitant avec celui-ci un volume de chargement de la boîte et une extrémité ouverte, les côtés latéraux comprenant, au voisinage de l'extrémité ouverte, un ensemble de découpes formant des volets rabattables.

En ce qui concerne le couvercle, selon une caractéristique de l'invention, celui-ci comprend un ensemble de rampes d'encliquetage agissant sur les volets pour l'encliquetage du couvercle sur le corps.

Avantageusement, le couvercle comprend un ensemble de surfaces de guidage alignées chacune avec une rampe d'encliquetage et adaptées pour solliciter l'extrémité de la paroi périphérique du couvercle dans le plan de ladite paroi.

De préférence, le corps est réalisé en carton, le couvercle pouvant être réalisé en matière plastique.

D'autres buts, caractéristiques et avantages de l'invention apparaîtront à la lecture de la description suivante, donnée uniquement à titre d'exemple non limitatif, et faite en référence aux dessins annexés sur lesquels :
- la figure 1 est une vue en perspective d'une boîte conforme à l'invention ;
- la figure 2 montre une vue de dessous du couvercle de la boîte de la figure 1 ;
- la figure 3 est une vue schématique de détail montrant les moyens d'encliquetage de la boîte de la figure 1 ; et
- les 4, 5 et 6 illustrent le principe de montage du couvercle sur le corps de la boîte de la figure 1.

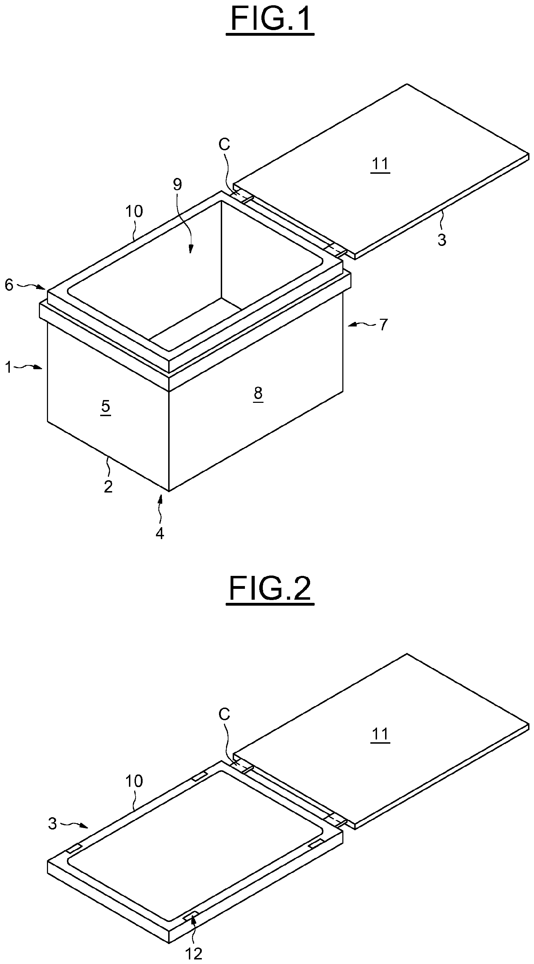

On se référera tout d'abord à la figure 1 qui illustre une vue en perspective d'une boîte selon l'invention, désignée par la référence numérique générale 1, dans une position normale d'utilisation.

Dans l'exemple de réalisation décrit, cette boîte 1 est destinée au conditionnement et à la distribution de bâtonnets ouatés, de type cotons-tiges. Mais, bien entendu, on ne sort pas du cadre de l'invention lorsqu'elle est destinée à recevoir d'autres types de produits, notamment, mais non exclusivement, des produits d'hygiène.

Comme on le voit, la boîte 1 comporte essentiellement un corps 2 et un couvercle 3 rapporté. Le corps et le couvercle peuvent être réalisés à partir de tout type de matériau convenant pour l'utilisation envisagée. Ils peuvent notamment être réalisés à partir de matériaux différents.

Le corps 2 est ici réalisé en carton ou à partir de tout autre matériau écologiquement acceptable, tandis que le couvercle 3 est réalisé en une pièce par moulage d'une matière thermoplastique, tel qu'un polymère thermoplastique.

Le corps 2 comporte un fond 4 et quatre faces latérales 5, 6, 7 et 8, seuls les côtés désignés par les références 5 et 8 étant visibles sur la figure 1 et une extrémité ouverte 9 par laquelle le couvercle vient se monter sur le corps. Il est réalisé par pliage et collage d'une ébauche unique en carton. Le fond et les côtés délimitent un volume de réception de bâtonnets accessible par l'ouverture 3 lorsque le couvercle est ouvert.

Le couvercle, quant à lui, comporte un cadre 10 venant se fixer par encliquetage sur le corps et un volet 11 relié au cadre 10 par une ou plusieurs charnières C de sorte que le volet puisse adopter une position ouverte visible sur la figure 1 rendant accessible l'intérieur du corps et une position fermée.

En se référant également à la figure 2, qui montre une vue de dessous du couvercle 3, c'est-à-dire de la face du couvercle destinée à être tournée vers l'intérieur du corps 2, le cadre et le corps sont dotés de moyens d'encliquetage complémentaires, tels que 12, schématiquement représentés sur la figure 2.

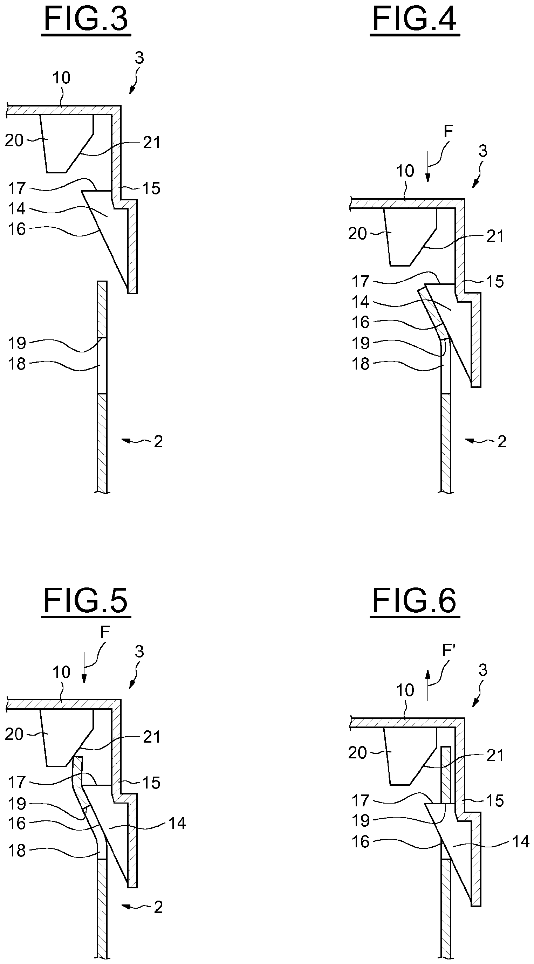

En se référant à la figure 3, ces moyens d'encliquetage comportent un ensemble de nervures internes, telles que 14, qui s'étendent perpendiculairement et vers l'intérieur à partir du bord 15 périphérique du cadre et qui forment une rampe 16 d'encliquetage réalisée à partir de l'extrémité libre du bord 15 jusqu'à une partie médiane où elle forme une surface de butée 17. Comme on le voit, dans cette zone, le corps comporte un épaulement optionnel.

Les faces latérales du corps 2 comprennent, quant à elles, des volets 18 qui coïncident avec les nervures 14 et qui sont réalisés à partir de trois découpes, à savoir deux latérales et une découpe transversale supérieure 19.

On voit encore sur la figure 3 que la face interne du capot 10 comporte une deuxième nervure axiale 20, c'est-à-dire une nervure qui s'étend parallèlement à la première nervure 14, en regard de celle-ci, et qui forme une surface inclinée 21 sur laquelle s'appuie l'extrémité supérieure d'un côté latéral de la boîte lors de l'encliquetage du cadre 10 sur le corps.

Comme on le voit, dans le mode de réalisation représenté, le couvercle et le corps comportent quatre moyens d'encliquetage, situés au voisinage des angles de la boîte.

Comme le montrent les figures 4, 5 et 6, lors de l'insertion du couvercle 3 sur le corps 2, lorsqu'on exerce un effort sur le couvercle 3 selon la flèche F, la rampe 16 assure une déformation localisée de l'extrémité supérieure du corps 2 jusqu'à ce que la première nervure 14 atteigne le volet 18 (figures 5 et 6).

On notera, comme le montre la figure 5, qu'avant que la première nervure 14 n'atteigne le volet 18, l'extrémité libre du corps vient en appui contre la surface de guidage 21 de la deuxième nervure 20 et est sollicitée vers le plan du côté latéral correspondant de la boîte pour contraindre de la sorte le volet 18 contre la rampe 16.

Dès que l'extrémité de la rampe 16 atteint le volet, celui-ci est rabattu pour dégager une ouverture dans laquelle s'engage la nervure 17 pour maintenir, de la sorte, le cadre 10 accroché sur le corps 2 et empêcher tout démontage intempestif du couvercle et du corps 2 (flèche F').

## Revendications

1. Boîte de conditionnement et de distribution de produits, notamment de produits d'hygiène, comprenant un corps (2) de forme parallélépipédique et un couvercle (3) monté sur le corps, ledit corps comporte un fond (4), une paroi périphérique comprenant un ensemble de côtés latéraux (5, 6, 7, 8) dressés à partir du fond et délimitant avec lui un volume de chargement de la boîte et une extrémité ouverte (9), les côtés latéraux comprenant, au voisinage de l'extrémité ouverte, un ensemble de découpes formant des volets rabattables (18), et ledit couvercle comportant des moyens d'encliquetage (12) destinés à coopérer avec des moyens d'encliquetage correspondants prévus sur le corps, lesdits moyens d'encliquetage (12) du couvercle comportant un ensemble de nervures internes (14) s'étendant perpendiculairement et vers l'intérieur à partir d'un bord (15) périphérique du couvercle et formant une rampe d'encliquetage agissant sur les volets (18) du corps pour l'encliquetage du couvercle sur le corps, **caractérisé en ce que** la face interne du couvercle comporte une deuxième nervure axiale (20) s'étendant parallèlement à la première nervure (14), en regard de celle-ci et formant une surface inclinée (21) sur laquelle s'appuie l'extrémité supérieure d'un côté latéral du corps lors de l'encliquetage du couvercle sur le corps.

2. Boîte selon la revendication 1, dans laquelle le corps et le couvercle sont réalisés à partir de matériaux différents.

3. Boîte selon la revendication 2, dans laquelle le couvercle comprend un ensemble de surfaces de guidage alignées chacune avec une rampe d'encliquetage et adaptées pour solliciter l'extrémité de la paroi périphérique du couvercle dans le plan de ladite paroi.

4. Boîte selon l'une quelconque des revendications 1 à 3, dans laquelle le corps est réalisé en carton.

5. Boîte selon l'une quelconque des revendications 1 à 4, dans laquelle le couvercle est réalisé en matière plastique.

6. Boîte selon l'une quelconque des revendications 1 à 5, dans laquelle le couvercle comporte un cadre (10) venant se fixer grâce aux moyens d'encliquetage sur le corps et un volet (11) relié au cadre par au moins une charnière (C), de sorte que le volet (11) puisse adopter une position ouverte rendant accessible l'intérieur du corps et une position fermée.

## Patentansprüche

1. Schachtel zur Verpackung und Verteilung von Produkten, insbesondere von Hygieneprodukten, umfassend einen quaderförmigen Körper (2) und einen Deckel (3), der auf den Körper montiert ist, wobei der Körper einen Boden (4), eine umlaufende Wand, aufweisend einen Satz lateraler Seitenflächen (5, 6, 7, 8), die ausgehend von dem Boden aufgestellt sind und mit ihm ein Ladevolumen der Schachtel abgrenzen, und ein offenes Ende (9) umfasst, wobei die lateralen Seitenflächen dem offenen Ende benachbart einen Satz Ausschnitte aufweisen, die klappbare Klappen (18) bilden, und der Deckel Einrastmittel (12) umfasst, die dazu bestimmt sind, mit entsprechenden Einrastmitteln zusammenzuwirken, die auf dem Körper vorgesehen sind, wobei die Einrastmittel (12) des Deckels einen Satz Innenrippen (14) umfassen, die sich senkrecht und nach innen ausgehend von einem Umfangsrand (15) des Deckels erstrecken und eine Einrastrampe bilden, die zum Einrasten des Deckels auf den Körper auf die Klappen (18) des Körpers wirkt, **dadurch gekennzeichnet, dass** die Innenseite des Deckels eine zweite axiale Rippe (20) umfasst, die sich parallel zu der ersten Rippe (14) gegenüber von dieser erstreckt und eine schräge Fläche (21) bildet, auf die das obere Ende einer lateralen Seitenfläche des Körpers beim Einrasten des Deckels auf den Körper drückt.

2. Schachtel nach Anspruch 1, wobei der Körper und der Deckel aus verschiedenen Materialien hergestellt sind.

3. Schachtel nach Anspruch 2, wobei der Deckel einen Satz Führungsflächen aufweist, die jeweils mit einer Einrastrampe ausgerichtet sind und angepasst sind, das Ende der umlaufenden Wand des Deckels in die Ebene der Wand vorzuspannen.

4. Schachtel nach einem der Ansprüche 1 bis 3, wobei der Körper aus Karton hergestellt ist.

5. Schachtel nach einem der Ansprüche 1 bis 4, wobei der Deckel aus Kunststoff hergestellt ist.

6. Schachtel nach einem der Ansprüche 1 bis 5, wobei der Deckel einen Rahmen (10), der mittels der Einrastmittel auf dem Körper befestigt wird, und eine Klappe (11) umfasst, die mit zumindest einem Scharnier (C) mit dem Rahmen verbunden ist, so dass die Klappe (11) eine offene Position, die das Innere des Körpers zugänglich macht, und eine geschlossene Position einnehmen kann.

## Claims

1. Box for packaging and dispensing products, particularly sanitary products, comprising a parallelepipedal body (2) and a lid (3) mounted on the body, said body comprising a base (4), a peripheral wall comprising a set of side walls (5, 6, 7, 8) rising from the base and delimiting therewith a loading volume of the box and an open end (9), the side walls comprising, close to the open end, a set of cutouts forming foldable flaps (18), and said lid comprising snap-fitting means (12) designed to engage with corresponding snap-fitting means provided on the body, said snap-fitting means (12) of the lid comprising a set of internal ribs (14) extending perpendicularly and inwards from a peripheral edge (15) of the lid and forming a snap-fitting ramp acting on the flaps (18) of the body in order to snap-fit the lid to the body, **characterized in that** the internal face of the lid comprises a second axial rib (20) extending parallel to the first rib (14), opposite the latter and forming an inclined surface (21) against which the upper end of a lateral side of the body presses during snap-fitting of the lid on the body.

2. Box according to Claim 1, in which the body and the lid are made of different materials.

3. Box according to Claim 2, in which the lid comprises a set of guiding surfaces each aligned with a snap-fitting ramp and designed to urge the end of the peripheral wall of the lid into the plane of said wall.

4. Box according to any one of Claims 1 to 3, in which the body is made of cardboard.

5. Box according to any one of Claims 1 to 4, in which the lid is made of plastic.

6. Box according to any one of Claims 1 to 5, in which the lid comprises a frame (10) which is secured, by virtue of the snap-fitting means, to the body and a flap (11) connected to the frame by at least one hinge (C) such that the flap (11) can adopt an open position allowing access to the interior of the body, and a closed position.
